# EUROPEAN PATENT APPLICATION

(11) **EP 4 492 825 A1**
(43) Date of publication of application: **15.01.2025**
(21) Application number: 24179553.3
(22) Date of filing: 03.06.2024
(51) Int. Cl.: H04R 25/00

(54) **HEARING AID AND COCHLEAR IMPLANT WIRELESS SYSTEM**

(30) Priority: 13.07.2023 US 202363513507 P
(71) Applicant: Starkey Laboratories, Inc., Eden Prairie, MN 55344 (US)
(72) Inventor: Achintya Kumar, Bhowmik, Cupertino, CA (US); Chaitanya, Jidge, Chanhassen, MN (US)
(74) Representative: Vossius & Partner Patentanwälte Rechtsanwälte mbB

(57) **Abstract**

Disclosed herein, among other things, are systems and methods for wireless communication with hearing devices. A method includes configuring, using a first hearing device configured to be worn or implanted in a first ear of a user, a first device parameter data. The method further includes transmitting, from the first hearing device via a wireless connection to a second hearing device configured to be worn or implanted in a second ear of the user, the first device parameter data. The method also includes receiving, at the second hearing device via the wireless connection, the first device parameter data from the first hearing device, and configuring the second hearing device for coordinated binaural operations with the first hearing device using the first device parameter data.

## Description

### CLAIM OF PRIORITY AND INCORPORATION BY REFERENCE

The present application claims the benefit under 35 U.S.C. §119(e) of U.S. Provisional Patent Application 63/513,507, filed July 13, 2023, the disclosure of which is hereby incorporated by reference herein in its entirety.

### TECHNICAL FIELD

This document relates generally to wireless communication systems and more particularly to systems and methods for wireless communications for hearing devices.

### BACKGROUND

Audio devices can be used to provide audible output to a user based on received wireless signals. Examples of audio devices include speakers and ear-wearable or implantable devices, also referred to herein as hearing devices. Example of hearing devices include hearing assistance devices or hearing instruments, including both prescriptive devices and non-prescriptive devices. Specific examples of hearing devices include, but are not limited to, hearing aids, cochlear implants, headphones, and earbuds.

Hearing aids are used to assist patients suffering hearing loss by transmitting amplified sounds to ear canals. In one example, a hearing aid is worn in and/or around a patient's ear. Hearing aids may include processors and electronics that improve the listening experience for a specific wearer or in a specific acoustic environment. Cochlear implants may also be used to assist patients suffering hearing loss by transmitting amplified sounds.

When a patient uses hearing devices of different types, it can be difficult to coordinate communications with and between the different device types. For example, a patient may have both hearing aid and cochlear type devices. Thus, there is a need in the art for systems and methods for wireless communications for differing types of hearing devices.

### SUMMARY

Disclosed herein, among other things, are systems and methods for wireless communication with hearing devices. A method includes configuring, using a first hearing device configured to be worn or implanted in a first ear of a user, first device parameter data. The method further includes transmitting, from the first hearing device via a wireless connection to a second hearing device configured to be worn or implanted in a second ear of the user, the first device parameter data. The method also includes receiving, at the second hearing device via the wireless connection, the first device parameter data from the first hearing device, and configuring the second hearing device for coordinated binaural operations with the first hearing device using the first device parameter data. In some examples, an external or central device may be used to pass parameter data between the first and second hearing devices.

Various aspects include a method for configuring hearing devices of different types to function as a binaural pair. The method includes receiving, at an external device, first device parameter data from a first hearing device configured to be worn or implanted in a first ear of a user. The method also includes receiving, at the external device, second device parameter data from a second hearing device configured to be worn or implanted in a second ear of the user, wherein the second hearing device and the first hearing device are of different device type. The method further includes configuring, by the external device, the first hearing device and the second hearing device to function as a binaural pair using the first device parameter data and the second device parameter data. The method may also include communicating, by the external device, with the first hearing device and the second hearing device as the binaural pair.

This Summary is an overview of some of the teachings of the present application and not intended to be an exclusive or exhaustive treatment of the present subject matter. Further details about the present subject matter are found in the detailed description and appended claims.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 illustrates a system for wireless communication between hearing devices and an external device, according to various examples of the present subject matter.
FIG. 2 illustrates a block diagram of a hearing device circuit, according to various examples of the present subject matter.
FIG. 3A illustrates a flow diagram of a method for wireless communications for hearing devices, according to various examples of the present subject matter.
FIG. 3B illustrates a flow diagram of a method for configuring hearing devices of different types to function as a binaural pair, according to various examples of the present subject matter.
FIG. 4 illustrates a block diagram of an example machine upon which any one or more of the techniques discussed herein may perform.

### DETAILED DESCRIPTION

The following detailed description of the present subject matter refers to subject matter in the accompanying drawings which show, by way of illustration, specific aspects and embodiments in which the present subject matter may be practiced. These embodiments are described in sufficient detail to enable those skilled in the art to practice the present subject matter. References to "an", "one", or "various" embodiments in this disclosure are not necessarily to the same embodiment, and such references contemplate more than one embodiment, including combinations of such embodiments. The following detailed description is demonstrative and not to be taken in a limiting sense. The scope of the present subject matter is defined by the appended claims, along with the full scope of legal equivalents to which such claims are entitled.

The present detailed description will discuss audio devices such as hearing devices and speakers. The description refers to hearing devices generally, which include earbuds, headsets, headphones and hearing assistance devices using the example of hearing aids and cochlear implants. Other hearing devices include, but are not limited to, those in this document. It is understood that their use in the description is intended to demonstrate the present subject matter, but not in a limited or exclusive or exhaustive sense.

When a patient uses hearing devices of different types, it can be difficult to coordinate communications with and between the different device types. For example, a patient may have both hearing aid and cochlear type devices. The present subject matter provides systems and methods for wireless communications for differing types of hearing devices used by a patient.

In various examples, a patient (or user) may have different levels or types of hearing loss in each ear, and thus may use a different type of hearing device in each ear. In one example, a user or patient has a hearing aid in or on one ear and a cochlear implant near the opposite ear. The present subject matter provides for configuring the hearing aid and the cochlear implant to work together wirelessly, by passing configuration data or timing data, such as hearing aid or cochlear implant parameters, back and forth between devices or through an external device to provide for timing synchronization to enable binaural functioning of the hearing and the cochlear implant, in this example. This subject matter enables the user to have full wireless functionality despite having two completely different hearing devices in opposite ears. The present subject matter can likewise enable full wireless functionality for different types of hearing aids in opposite ears, or different types of implant devices in opposite ears, etc. Once the configuration data or parameter data is exchanged, such as by programming via an external device, the external device may control or stream content to the hearing devices simultaneously, in various examples.

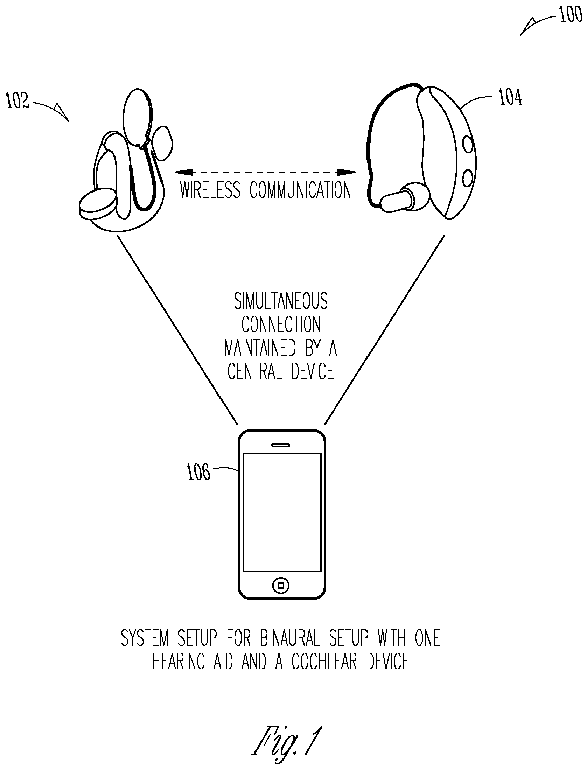

FIG. 1 illustrates a system 100 for wireless communication between hearing devices 102, 104 and an external device 106, according to various examples of the present subject matter. In the depicted embodiment, a user has a cochlear implant type of hearing device 102 in a first ear and a hearing aid type of hearing device 104 in a second ear. Using the methods of the present subject matter the external device 106, in this case a smartphone, may connect simultaneously with the cochlear implant 102 and the hearing aid 104 for control and streaming of audio or other information. Other types of hearing devices may be used without departing from the present subject matter. Examples include, but are not limited to, ear buds, bone conduction devices, headphones, hearing assistive devices, and the like. In addition, other types of external devices may be used without departing from the scope of the present subject matter. Examples include, but are not limited to, other hearing devices of other users, a hearing device host device, a hearing device programming device, an assistive listening device (ALD), a smartphone, a personal computer, a tablet, an audio streaming device, and other devices capable of communicating with hearing devices wirelessly.

According to various examples, the present subject matter gathers key configuration information that needs to be programmed for a hearing device, and uses the configuration information to match the configuration data from another hearing device of different make, model and/or type, to ensure the devices complement each other and can provide full wireless programming, control and streaming as if the devices were a matched set. In some examples, an external device is programmed with the configuration information of each hearing device to enable the external device to communicate simultaneously or substantially simultaneously so that the hearing devices can function as a binary pair for control and streaming.

In various examples, the present system provides wireless techniques for making different hearing device types work together as a binaural pair. The present subject matter may enable high-precision audio synchronization such that, for example, low frequencies from the hearing aid and high frequencies from the cochlear implant arrive together. In some examples, the present subject matter may provide for the hearing devices to be used together for streaming, audio balance between hearing aid and cochlear implant devices, noise management (such as using filters for comfort), and/or clarity enhancement. In various examples, the present subject matter provides for ear-to-ear communication, such as a left-to-right "cross" feature where a person has a functional hearing aid in right ear but not the left ear.

According to various examples, the hearing devices share their respective addresses, such as a Bluetooth^{®} addresses, with each other or with a central or external device. Other parameters may be used or included or shared without departing from the scope of the present subject matter. In some examples, the hearing devices share or exchange key parameters for out-of-band communication and can be programmed using the exchanged parameters to function as a binaural pair, such as programming respective devices to function as a left and right complementary component of the pair.

In some examples, after the mis-matched hearing devices (such as a hearing aid in one ear and a cochlear implant in the other ear) share the parameter information or data (such as a data string or data packet or other type of data), the hearing devices may receive commands to be controlled as a binaural pair, and further may receive streaming content from a central external device as a binaural pair. The devices further may synchronize reception or transmission from ear-to-ear with respect to audio applications. In addition, the devices may activate more aggressive noise suppression, such as in a binaural streaming mode. The devices may also use an audio-crossing mechanism based on the acoustic environment, in various examples. After using the methods of the present subject matter, the mis-matched hearing devices may communicate as a pair with any central device and any related audio application, in various examples. Further, the present subject matter may enable the hearing devices to communicate ear-to-ear as a binaural pair, controlling audio delivery in a stereo system, for example.

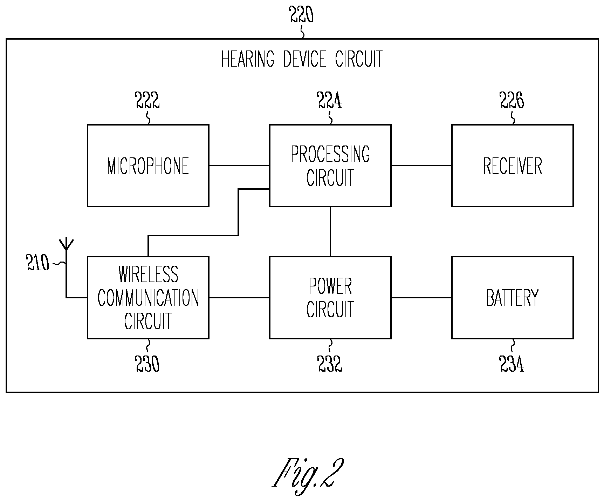

FIG. 2 illustrates a block diagram of a hearing device circuit, according to various examples of the present subject matter. Hearing device circuit 220 represents an example of portions of a hearing device and includes a microphone 222, a wireless communication circuit 230, an antenna 210, a processing circuit 224, a receiver (speaker) 226, a battery 234, and a power circuit 232. Microphone 222 receives sounds from the environment of the hearing device user (wearer of the hearing device). Wireless communication circuit 230 communicates with an external device wirelessly using antenna 210, including receiving programming codes, streamed audio signals, and/or other audio signals and transmitting programming codes, audio signals, and/or other signals. Examples of the external devices include other hearing devices of other users, another hearing device of a pair (matched or mismatched) of hearing devices for the same wearer, a hearing device host device, an ALD, an audio streaming device, a smartphone, and other devices capable of communicating with hearing devices wirelessly. Processing circuit 224 controls the operation of hearing device using the programming codes and processes the sounds received by microphone 222 and/or the audio signals received by wireless communication circuit 230 to produce output sounds. Receiver 226 transmits output sounds to an ear canal of the hearing device wearer. Battery 234 and power circuit 232 constitute the power source for the operation of hearing device circuit 220. In one example, power circuit 232 can include a power management circuit. In another alternative or additional example, battery 234 can include a rechargeable battery, and power circuit 232 can include a recharging circuit for recharging the rechargeable battery. The hearing device circuit 224, or a partial version of this circuit, may be included in an ear bud, headphones, a hearing aid, a cochlear implant or other hearing device, in various examples.

In various examples, at least one of the mismatched hearing devices includes a connection to a smartphone application. The smartphone application is configured to be used to control the hearing devices, in some examples. In some examples, at least one of the hearing devices includes a voice control configured to be used to control the hearing devices. In various examples, at least one of the hearing devices is a hearing assistance device, such as a hearing aid. In various examples, at least one of the hearing devices is an implantable hearing assistance device, such as a cochlear implant.

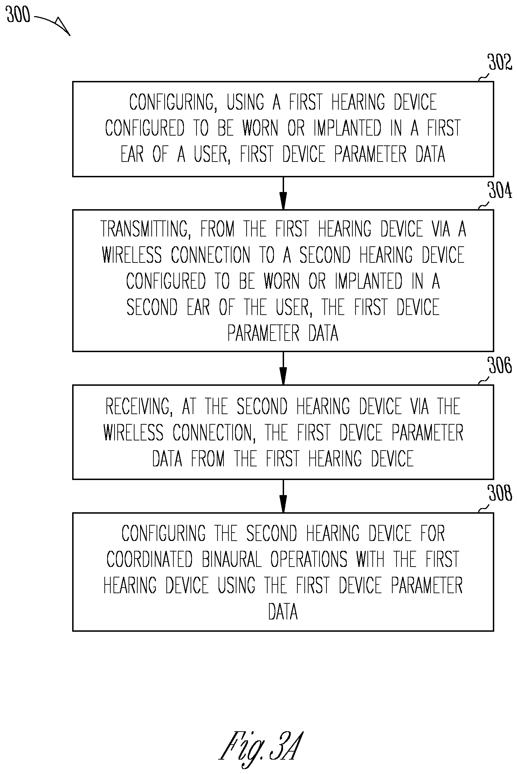

FIG. 3A illustrates a flow diagram of a method for wireless communications for hearing devices, according to various examples of the present subject matter. The method 300 includes configuring, using a first hearing device configured to be worn or implanted in a first ear of a user, first device parameter data, at step 302. The method further includes transmitting, from the first hearing device via a wireless connection to a second hearing device configured to be worn or implanted in a second ear of the user, the first device parameter data, at step 304. At step 306, the method also includes receiving, at the second hearing device via the wireless connection, the first device parameter data from the first hearing device. At step 308, the method includes configuring the second hearing device for coordinated binaural operations with the first hearing device using the first device parameter data. In some examples, an external or central device may be used to pass parameter data between the first and second hearing devices.

In various examples, the first hearing device may include an ear bud, headphones, a hearing aid, a cochlear implant, or other ear-wearable or implantable device. In various examples, the second hearing device may include an ear bud, headphones, a hearing aid, a cochlear implant, or other ear-wearable or implantable device. The wireless connection includes a Bluetooth^{®} compatible wireless connection, such as Bluetooth^{®} Low Energy (BLE), in various examples. In various examples, the first device parameter data includes one or more of a first device configuration, first device timing transmission parameters, first device timing receipt parameters, first device wireless communication circuit type, or first device antenna type. According to various examples, the first and second device have interchangeable roles, and hence the second device may configure and transmit a second device parameter data to the first device to accomplish the coordinated binaural operations.

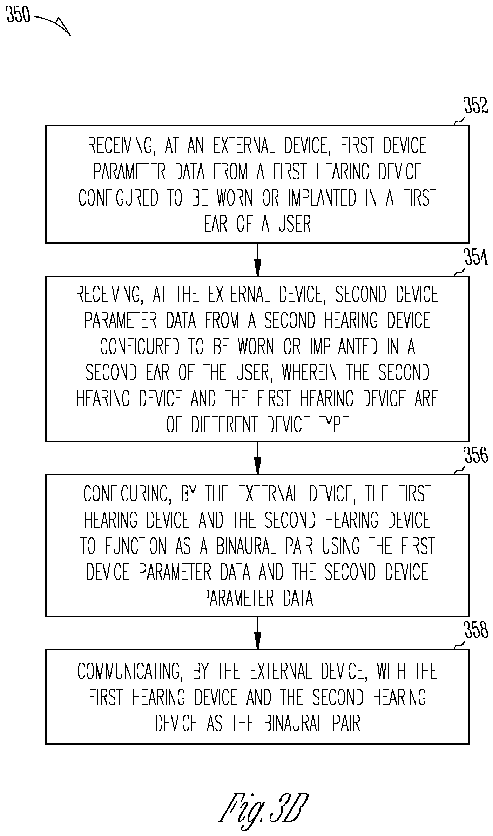

FIG. 3B illustrates a flow diagram of a method for configuring hearing devices of different types to function as a binaural pair, according to various examples of the present subject matter. The method 350 includes receiving, at an external device, first device parameter data from a first hearing device configured to be worn or implanted in a first ear of a user, at step 352. At step 354, the method also includes receiving, at the external device, second device parameter data from a second hearing device configured to be worn or implanted in a second ear of the user, wherein the second hearing device and the first hearing device are of different device type. The method further includes configuring, by the external device, the first hearing device and the second hearing device to function as a binaural pair using the first device parameter data and the second device parameter data, at step 356. The method may also include communicating, by the external device, with the first hearing device and the second hearing device as the binaural pair, at step 358.

In various examples, the external device includes a hearing device programmer. In other examples, the external device includes one or more of a smartphone, a personal computer, a smartwatch, or a tablet. The first hearing device is a hearing aid and the second hearing device is a cochlear implant, in one example. Other types of hearing devices may be used without departing from the scope of the present subject matter. The term device type may refer to function, manufacturer, size, shape or other distinguishing characteristic of respective hearing devices.

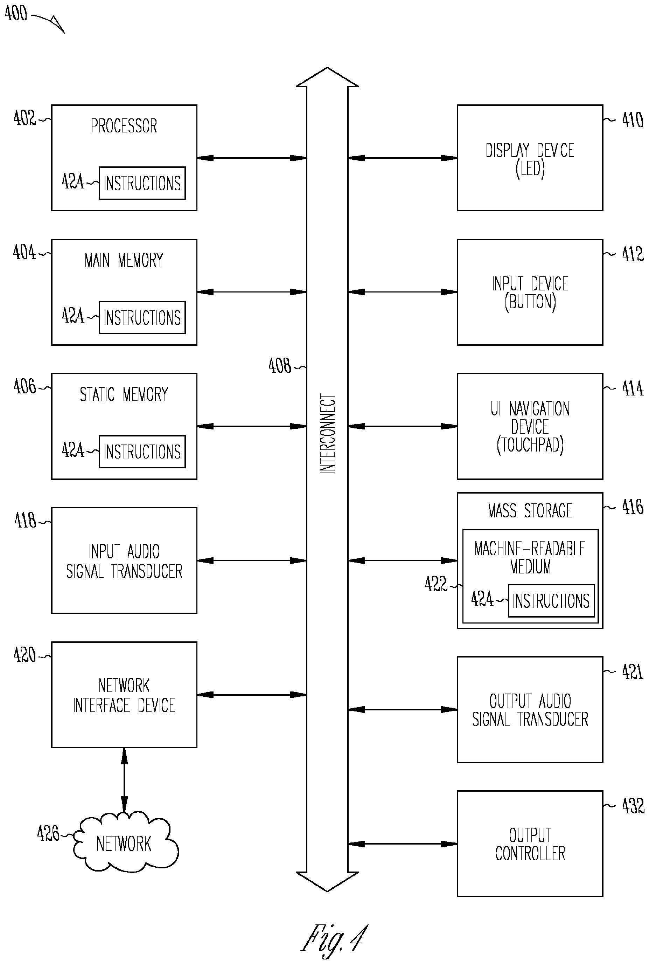

FIG. 4 illustrates a block diagram of an example machine 400 upon which any one or more of the techniques (e.g., methodologies) discussed herein may perform. In alternative examples, the machine 400 may operate as a standalone device or may be connected (e.g., networked) to other machines. In a networked deployment, the machine 400 may operate in the capacity of a server machine, a client machine, or both in server-client network environments. In an example, the machine 400 may act as a peer machine in peer-to-peer (P2P) (or other distributed) network environment. The machine 400 may be a personal computer (PC), a tablet PC, a set-top box (STB), a personal digital assistant (PDA), a mobile telephone, a hearing device, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 400 may include a hardware processor 402 (e.g., a central processing unit (CPU), a graphics processing unit (GPU), a hardware processor core, or any combination thereof), a main memory 404 and a static memory 406, some or all of which may communicate with each other via an interlink (e.g., bus) 408. The machine 400 may further include a display unit 410, an alphanumeric input device 412 (e.g., a keyboard), and a user interface (UI) navigation device 414 (e.g., a mouse). In an example, the display unit 410, input device 412 and UI navigation device 414 may be a touch screen display. The machine 400 may additionally include a storage device (e.g., drive unit) 416, one or more input audio signal transducers 418 (e.g., microphone), a network interface device 420, and one or more output audio signal transducer 421 (e.g., speaker). The machine 400 may include an output controller 432, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 416 may include a machine readable medium 422 on which is stored one or more sets of data structures or instructions 424 (e.g., software) embodying or utilized by any one or more of the techniques or functions described herein. The instructions 424 may also reside, completely or at least partially, within the main memory 404, within static memory 406, or within the hardware processor 402 during execution thereof by the machine 400. In an example, one or any combination of the hardware processor 402, the main memory 404, the static memory 406, or the storage device 416 may constitute machine readable media.

While the machine readable medium 422 is illustrated as a single medium, the term "machine readable medium" may include a single medium or multiple media (e.g., a centralized or distributed database, and/or associated caches and servers) configured to store the one or more instructions 424.

The term "machine readable medium" may include any medium that is capable of storing, encoding, or carrying instructions for execution by the machine 400 and that cause the machine 400 to perform any one or more of the techniques of the present disclosure, or that is capable of storing, encoding or carrying data structures used by or associated with such instructions. Non-limiting machine-readable medium examples may include solid-state memories, and optical and magnetic media. In an example, a massed machine-readable medium comprises a machine-readable medium with a plurality of particles having invariant (e.g., rest) mass. Accordingly, massed machine-readable media are not transitory propagating signals. Specific examples of massed machine-readable media may include: nonvolatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 424 may further be transmitted or received over a communications network 426 using a transmission medium via the network interface device 420 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 420 may include one or more physical jacks (e.g., Ethernet, coaxial, or phone jacks) or one or more antennas to connect to the communications network 426. In an example, the network interface device 420 may include a plurality of antennas to communicate wirelessly using at least one of single-input multiple-output (SIMO), multiple-input multiple-output (MIMO), or multiple-input single-output (MISO) techniques. The term "transmission medium" shall be taken to include any intangible medium that is capable of storing, encoding, or carrying instructions for execution by the machine 400, and includes digital or analog communications signals or other intangible medium to facilitate communication of such software.

Various examples of the present subject matter support wireless communications with a hearing device. In various examples the wireless communications may include standard or nonstandard communications. Some examples of standard wireless communications include link protocols including, but not limited to, Bluetooth^{™}, Bluetooth^{™} Low Energy (BLE), Auracast, IEEE 802.11 (wireless LANs), 802.15 (WPANs), 802.16 (WiMAX), cellular protocols including, but not limited to CDMA and GSM, ZigBee, and ultra-wideband (UWB) technologies. Such protocols support radio frequency communications and some support infrared communications while others support NFMI. Although the present system is demonstrated as a radio system, it is possible that other forms of wireless communications may be used such as ultrasonic, optical, infrared, and others. It is understood that the standards which may be used include past and present standards. It is also contemplated that future versions of these standards and new future standards may be employed without departing from the scope of the present subject matter.

The wireless communications support a connection from other devices. Such connections include, but are not limited to, one or more mono or stereo connections or digital connections having link protocols including, but not limited to 802.3 (Ethernet), 802.4, 802.5, USB, SPI, PCM, ATM, Fibre-channel, Firewire or 1394, InfiniBand, or a native streaming interface. In various examples, such connections include all past and present link protocols. It is also contemplated that future versions of these protocols and new future standards may be employed without departing from the scope of the present subject matter.

Hearing assistance devices typically include at least one enclosure or housing, a microphone, hearing assistance device electronics including processing electronics, and a speaker or "receiver." Hearing assistance devices may include a power source, such as a battery. In various examples, the battery is rechargeable. In various examples multiple energy sources are employed. It is understood that in various examples the microphone is optional. It is understood that in various examples the receiver is optional. It is understood that variations in communications protocols, antenna configurations, and combinations of components may be employed without departing from the scope of the present subject matter. Antenna configurations may vary and may be included within an enclosure for the electronics or be external to an enclosure for the electronics. Thus, the examples set forth herein are intended to be demonstrative and not a limiting or exhaustive depiction of variations.

It is understood that digital hearing assistance devices include a processor. In digital hearing assistance devices with a processor, programmable gains may be employed to adjust the hearing assistance device output to a wearer's particular hearing impairment. The processor may be a digital signal processor (DSP), microprocessor, microcontroller, other digital logic, or combinations thereof. The processing may be done by a single processor, or may be distributed over different devices. The processing of signals referenced in this application may be performed using the processor or over different devices. Processing may be done in the digital domain, the analog domain, or combinations thereof. Processing may be done using subband processing techniques. Processing may be done using frequency domain or time domain approaches. Some processing may involve both frequency and time domain aspects. For brevity, in some examples drawings may omit certain blocks that perform frequency synthesis, frequency analysis, analog-to-digital conversion, digital-to-analog conversion, amplification, buffering, and certain types of filtering and processing. In various examples of the present subject matter the processor is adapted to perform instructions stored in one or more memories, which may or may not be explicitly shown. Various types of memory may be used, including volatile and nonvolatile forms of memory. In various examples, the processor or other processing devices execute instructions to perform a number of signal processing tasks. Such examples may include analog components in communication with the processor to perform signal processing tasks, such as sound reception by a microphone, or playing of sound using a receiver (i.e., in applications where such transducers are used). In various examples of the present subject matter, different realizations of the block diagrams, circuits, and processes set forth herein may be created by one of skill in the art without departing from the scope of the present subject matter.

It is further understood that different hearing devices may embody the present subject matter without departing from the scope of the present disclosure. The devices depicted in the figures are intended to demonstrate the subject matter, but not necessarily in a limited, exhaustive, or exclusive sense. It is also understood that the present subject matter may be used with a device designed for use in the right ear or the left ear or both ears of the wearer.

The present subject matter is demonstrated for hearing devices, including hearing assistance devices, including but not limited to, behind-the-ear (BTE), in-the-ear (ITE), in-the-canal (ITC), receiver-in-canal (RIC), invisible-in-canal (IIC) or completely-in-the-canal (CIC) type hearing assistance devices. It is understood that behind-the-ear type hearing assistance devices may include devices that reside substantially behind the ear or over the ear. Such devices may include hearing assistance devices with receivers associated with the electronics portion of the behind-the-ear device, or hearing assistance devices of the type having receivers in the ear canal of the user, including but not limited to receiver-in-canal (RIC) or receiver-in-the-ear (RITE) designs. The present subject matter may also be used in hearing assistance devices generally, such as cochlear implant type hearing devices. The present subject matter may also be used in deep insertion devices having a transducer, such as a receiver or microphone. The present subject matter may be used in bone conduction hearing devices, in some examples. The present subject matter may be used in devices whether such devices are standard or custom fit and whether they provide an open or an occlusive design. It is understood that other hearing devices not expressly stated herein may be used in conjunction with the present subject matter.

### Other Notes and Examples

Example 1 is a method including configuring, using a first hearing device configured to be worn or implanted in a first ear of a user, first device parameter data, transmitting, from the first hearing device via a wireless connection to a second hearing device configured to be worn or implanted in a second ear of the user, the first device parameter data, receiving, at the second hearing device via the wireless connection, the first device parameter data from the first hearing device, and configuring the second hearing device for coordinated binaural operations with the first hearing device using the first device parameter data.

In Example 2, the subject matter of Example 1 optionally further includes using an external or central device to transmit the first device parameter data between the first hearing device and the second hearing device.

In Example 3, the subject matter of Example 1 optionally includes wherein at least one of the first hearing device and the second hearing device is a hearing aid.

In Example 4, the subject matter of Example 1 optionally includes wherein at least one of the first hearing device and the second hearing device is a cochlear implant.

In Example 5, the subject matter of Example 1 optionally includes wherein the wireless connection includes a Bluetooth^{®} compatible wireless connection.

In Example 6, the subject matter of Example 1 optionally includes wherein the first device parameter data includes a first device configuration.

In Example 7, the subject matter of Example 1 optionally includes wherein the first device parameter data includes a first device timing transmission parameter.

In Example 8, the subject matter of Example 1 optionally includes wherein the first device parameter data includes a first device timing receipt parameter.

In Example 9, the subject matter of Example 1 optionally includes wherein the first device parameter data includes a first device wireless communication circuit type.

In Example 10, the subject matter of Example 1 optionally includes wherein the first device parameter data includes a first device antenna type.

Example 11 is a method including receiving, at an external device, first device parameter data from a first hearing device configured to be worn or implanted in a first ear of a user, receiving, at the external device, second device parameter data from a second hearing device configured to be worn or implanted in a second ear of the user, wherein the second hearing device and the first hearing device are of different device type, configuring, by the external device, the first hearing device and the second hearing device to function as a binaural pair using the first device parameter data and the second device parameter data, and communicating, by the external device, with the first hearing device and the second hearing device as the binaural pair.

In Example 12, the subject matter of Example 11 optionally includes wherein the external device includes a hearing device programmer.

In Example 13, the subject matter of Example 11 optionally includes wherein the external device includes a smartphone.

In Example 14, the subject matter of Example 11 optionally includes wherein the external device includes a personal computer.

In Example 15, the subject matter of Example 11 optionally includes wherein the external device includes a smartwatch.

In Example 16, the subject matter of Example 11 optionally includes wherein the external device includes a tablet.

In Example 17, the subject matter of Example 11 optionally includes wherein the first hearing device is a hearing aid.

In Example 18, the subject matter of Example 17 optionally includes wherein the second hearing device is a cochlear implant.

In Example 19, the subject matter of Example 11 optionally includes wherein the first device parameter data includes a first device configuration.

In Example 20, the subject matter of Example 11 optionally includes wherein the second device parameter data includes a second device configuration.

Example 21 is at least one machine-readable medium including instructions that, when executed by processing circuitry, cause the processing circuitry to perform operations to implement of any of Examples 1-20.

Example 22 is an apparatus comprising means to implement of any of Examples 1-20.

Example 23 is a system to implement of any of Examples 1-20.

Example 24 is a method to implement of any of Examples 1-20.

This application is intended to cover adaptations or variations of the present subject matter. It is to be understood that the above description is intended to be illustrative, and not restrictive. The scope of the present subject matter should be determined with reference to the appended claims, along with the full scope of legal equivalents to which such claims are entitled.

The application relates, inter alia, to the following aspects:
1. A method, comprising:
   configuring, using a first hearing device configured to be worn or implanted in a first ear of a user, first device parameter data;
   transmitting, from the first hearing device via a wireless connection to a second hearing device configured to be worn or implanted in a second ear of the user, the first device parameter data;
   receiving, at the second hearing device via the wireless connection, the first device parameter data from the first hearing device; and
   configuring the second hearing device for coordinated binaural operations with the first hearing device using the first device parameter data.
2. The method of aspect 1, further comprising using an external or central device to transmit the first device parameter data between the first hearing device and the second hearing device.
3. The method of aspect 1 or 2, wherein at least one of the first hearing device and the second hearing device is a hearing aid.
4. The method of any preceding aspect, wherein at least one of the first hearing device and the second hearing device is a cochlear implant.
5. The method of any preceding aspect, wherein the wireless connection includes a Bluetooth^{®} compatible wireless connection.
6. The method of any preceding aspect, wherein the first device parameter data includes a first device configuration.
7. The method of any preceding aspect, wherein the first device parameter data includes a first device timing transmission parameter.
8. The method of any preceding aspect, wherein the first device parameter data includes a first device timing receipt parameter.
9. The method of any preceding aspect, wherein the first device parameter data includes a first device wireless communication circuit type.
10. The method of any preceding aspect, wherein the first device parameter data includes a first device antenna type.
11. A method, comprising:
   receiving, at an external device, first device parameter data from a first hearing device configured to be worn or implanted in a first ear of a user;
   receiving, at the external device, second device parameter data from a second hearing device configured to be worn or implanted in a second ear of the user, wherein the second hearing device and the first hearing device are of different device type;
   configuring, by the external device, the first hearing device and the second hearing device to function as a binaural pair using the first device parameter data and the second device parameter data; and
   communicating, by the external device, with the first hearing device and the second hearing device as the binaural pair.
12. The method of aspect 11, wherein the external device includes a hearing device programmer.
13. The method of aspect 11 or 12, wherein the external device includes a smartphone.
14. The method of aspect 11 or 12, wherein the external device includes a personal computer.
15. The method of aspect 11 or 12, wherein the external device includes a smartwatch.
16. The method of aspect 11 or 12, wherein the external device includes a tablet.
17. The method of any of claims 11 to 16, wherein the first hearing device is a hearing aid.
18. The method of any of claims 11 to 17, wherein the second hearing device is a cochlear implant.
19. The method of any of claims 11 to 18, wherein the first device parameter data includes a first device configuration.
20. The method of any of claims 11 to 19, wherein the second device parameter data includes a second device configuration.

## Claims

1. A method, comprising:
configuring, using a first hearing device configured to be worn or implanted in a first ear of a user, first device parameter data;
transmitting, from the first hearing device via a wireless connection to a second hearing device configured to be worn or implanted in a second ear of the user, the first device parameter data;
receiving, at the second hearing device via the wireless connection, the first device parameter data from the first hearing device; and
configuring the second hearing device for coordinated binaural operations with the first hearing device using the first device parameter data.

2. The method of claim 1, further comprising using an external or central device to transmit the first device parameter data between the first hearing device and the second hearing device.

3. The method of claim 1 or 2, wherein at least one of the first hearing device and the second hearing device is a hearing aid.

4. The method of any preceding claim, wherein at least one of the first hearing device and the second hearing device is a cochlear implant.

5. The method of any preceding claim, wherein the wireless connection includes a Bluetooth^{®} compatible wireless connection.

6. The method of any preceding claim, wherein the first device parameter data includes a first device configuration.

7. The method of any preceding claim, wherein the first device parameter data includes a first device timing transmission parameter.

8. The method of any preceding claim, wherein the first device parameter data includes a first device timing receipt parameter.

9. The method of any preceding claim, wherein the first device parameter data includes a first device wireless communication circuit type.

10. The method of any preceding claim, wherein the first device parameter data includes a first device antenna type.

11. A method, comprising:
receiving, at an external device, first device parameter data from a first hearing device configured to be worn or implanted in a first ear of a user;
receiving, at the external device, second device parameter data from a second hearing device configured to be worn or implanted in a second ear of the user, wherein the second hearing device and the first hearing device are of different device type;
configuring, by the external device, the first hearing device and the second hearing device to function as a binaural pair using the first device parameter data and the second device parameter data; and
communicating, by the external device, with the first hearing device and the second hearing device as the binaural pair.

12. The method of claim 11, wherein the external device includes a hearing device programmer.

13. The method of claim 11 or 12,
wherein the external device includes a smartphone; or
wherein the external device includes a personal computer; or
wherein the external device includes a smartwatch; or
wherein the external device includes a tablet.

14. The method of any of claims 11 to 13,
wherein the first hearing device is a hearing aid; and/or
wherein the second hearing device is a cochlear implant.

15. The method of any of claims 11 to 14,
wherein the first device parameter data includes a first device configuration; and/or
wherein the second device parameter data includes a second device configuration.
